# FASCICULE DE BREVET EUROPEEN

(11) **EP 1 924 230 B1**
(45) Date de publication et mention de la délivrance du brevet: **07.01.2009**
(21) Numéro de dépôt: 06808119.9
(22) Date de dépôt: 13.09.2006
(51) Int. Cl.: A61F 9/06

(54) **Perfectionnement aux casques de protection qui comportent un oculaire amovible et une coiffe à rapporter sur un cadre monté**
Verbesserungen für Schutzhelme umfassend ein entfernbares optisches Augenteil und eine Klappe, die auf einem Rahmen montiert ist, der drehbar auf einem Kopfband montiert ist.
Improvements to protective helmets comprising a removable optical eyepiece and a cap to be mounted on a frame pivotably mounted on a headband

(30) Priorité: 13.09.2005 FR 0509332
(43) Date de publication de la demande: 28.05.2008
(73) Titulaire: Sperian Respiratory Protection France, 93420 Villepinte (FR)
(72) Inventeur: THUON, Sébastien, F-69210 Villeurbanne (FR); YAOUANQ, Fabrice, F-02600 Mortefontaine (FR)
(74) Mandataire: Ahner, Francis
(86) Numéro de dépôt international: PCT/FR2006/002093
(87) Numéro de publication internationale: WO 2007/031641

(56) Documents cités:
- BE-A- 535 485
- US-A- 3 259 908
- US-A- 3 668 705
- US-A1- 2004 064 866
- US-A1- 2005 010 992
- US-A1- 2005 015 860

## Description

L'invention concerne les perfectionnements aux casques de protection (communément appelés « coiffes ») qui comportent un oculaire amovible et une coiffe à rapporter sur un cadre qui pivote sur un bandeau serre-tête.

On connaît de tels casques de protection qui comportent un cadre rigide qui pivote sur deux pivots latéraux d'un bandeau serre-tête de façon à pouvoir être basculé vers le haut pour dégager le visage ou rabattu en position de service, ce cadre déterminant une ouverture faciale et le casque comportant un oculaire devant l'ouverture faciale et une coiffe souple pour couvrir le sommet de la tête, une partie des côtés du visage et le bas du visage.

Ces casques peuvent présenter des défauts d'étanchéité à l'interface de l'oculaire, du cadre et de la coiffe.

Le document US 2005/0010992 décrit un casque de protection comportant un cadre rigide et un oculaire qui peut être fixé à une coiffe par une fixation étanche qui fait le tour de l'oculaire. L'oculaire peut être fixé au cadre par un système de clips ou des moyens magnétiques.

La présente invention vise à assurer une étanchéité plus sure à cet endroit critique tout en ayant un montage très simple de l'oculaire sur le cadre.

On y parvient selon l'invention, en fixant la coiffe directement à l'oculaire, par une fixation étanche, et en munissant l'oculaire d'encoches latérales pour le montage à pivotement de l'oculaire sur les axes de rotation du cadre sur le bandeau serre-tête.

De préférence, l'oculaire est également muni d'un moyen de fixation de l'oculaire sur une partie inférieure du cadre conformée en mentonnière.

Il est ainsi possible d'assurer à la fabrication une étanchéité parfaite entre l'oculaire et la coiffe.

On a déjà proposé de fixer directement une coiffe sur une visière, mais pour constituer une simple cagoule qui n'est pas conçue pour être rapportée sur un cadre.

On décrira ci-après un mode de réalisation conforme à l'invention, en référence aux figures du dessin joint sur lequel :

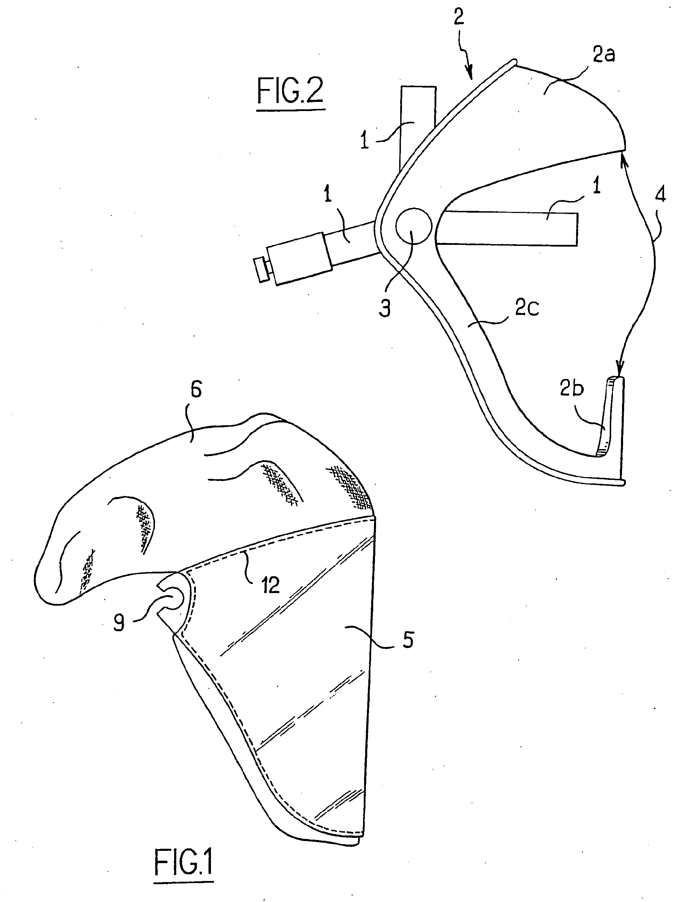

- la figure 1 est une vue latérale du sous-ensemble constitué par l'oculaire et la coiffe ;
- la figure 2 est une vue latérale du cadre et du serre-tête ;
- la figure 3 est une vue de l'oculaire à plat ;
- la figure 4 est une vue de détail du montage du sous-ensemble sur les pivots du bandeau ;
- la figure 5 est une vue de détail du montage à clip du sous-ensemble sur le cadre ;
- la figure 6 montre des vues successives de montage du sous-ensemble sur le casque ;
- la figure 7 représente une.variante de réalisation du cadre du casque, et
- la figure 8 est une vue de détails en coupe schématique d'une réalisation d'un pivot du bandeau serre-tête.

Le casque représenté sur les figures comprend :
- un bandeau serre-tête (1),
- un cadre (2) monté à pivotement sur le bandeau autour de pivots latéraux (3) situés sensiblement au niveau des oreilles et qui détermine une ouverture faciale (4);
- un sous-ensemble composé d'un oculaire (5) et d'une coiffe (6).

Le cadre (2) venu de moulage en matériau de synthèse, comporte une partie supérieure (2a) qui est conformée pour couvrir l'avant-tête, une partie inférieure (2b), conformée pour constituer une mentonnière (8), et deux parties latérales (2c) qui couvrent partiellement les côtés du visage et servent de liaison entre les parties supérieure et inférieure du casque et qui sont montées à pivotement sur les pivots (3) du bandeau serre-tête (1).

Les trois parties (2a, 2b, 2c) du cadre coopèrent pour déterminer une ouverture faciale (4).

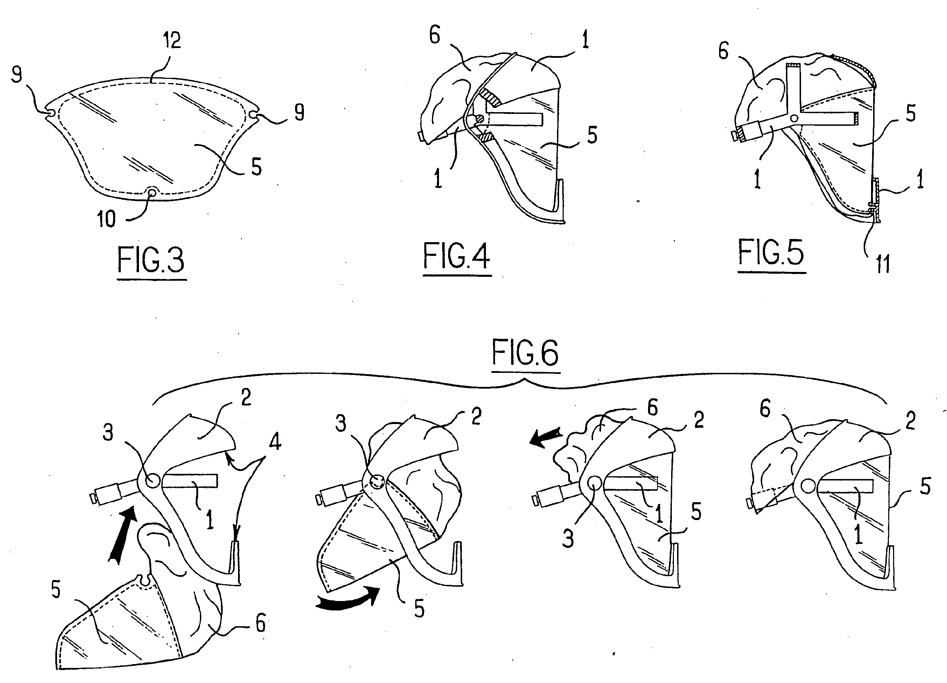

L'oculaire (5), en matériau semi-rigide peut être mis à plat (figure 3) et il présente deux encoches latérales (9) en sorte que l'oculaire courbé à la main peut être enclenché latéralement par les encoches sur les pivots (3), ce qui le maintient courbé (figure 4) devant l'ouverture faciale (4).

Ce maintien peut être renforcé par enclenchement d'un trou (10) prévu sur le bord inférieur de l'oculaire sur un clip (11) prévu sur la mentonnière du cadre (figure 5).

La coiffe (6) en nappe souple, est fixée sur tout le pourtour de l'oculaire par une fixation étanche (12), par exemple une couture ou un collage, qui contourne les encoches (9) et le trou (10).

Le sous-ensemble constitué par l'oculaire et la coiffe attenante s'insère par en dessous entre le cadre et le bandeau comme on le voit sur la figure 6, jusqu'à être monté sur les pivots, basculé vers le haut et fixé à la mentonnière.

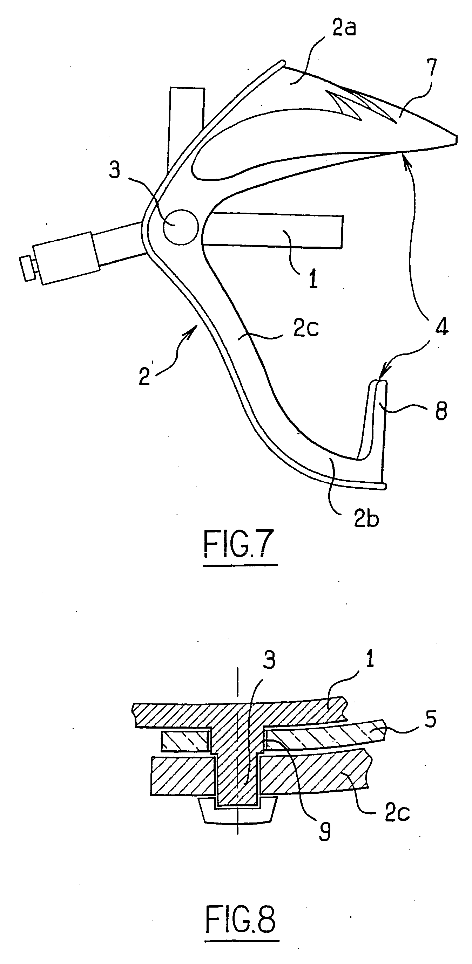

La figure 7 représente une variante de réalisation du cadre (2) dans laquelle le cadre présente au-dessus de l'emplacement (4) de l'oculaire, une visière (7) venue de moulage avec le cadre.

## Revendications

1. Casque de protection comportant un cadre rigide (2) qui pivote sur deux pivots latéraux (3) d'un bandeau serre-tête (1), de façon à pouvoir être basculé vers le haut pour dégager le visage ou rabattu en position de service, ce cadre déterminant une ouverture faciale (4) et le casque comportant un oculaire (5) devant l'ouverture faciale et une coiffe souple (6) pour couvrir le sommet de la tête, une partie des côtés du visage et le bas du visage, la coiffe étant fixée à l'oculaire par une fixation étanche (12) qui fait sensiblement le tour de l'oculaire et l'oculaire présentant deux encoches latérales (9) pour le montage de l'oculaire sur les pivots latéraux (3) du bandeau.

2. Casque de protection selon la revendication 1, dont l'oculaire (5) présente en partie basse un moyen de fixation (10) de l'oculaire à la partie basse (2b) du cadre.

3. Casque de protection selon la revendication 2 dont ledit moyen de fixation (10) est un trou.

4. Casque de protection selon la revendication 2 dont la partie basse (2b) du cadre comporte une mentonnière (8) pourvue d'un moyen (11) pour la fixation de l'oculaire.

5. Casque selon les revendications 3 et 4 dont le moyen de fixation dont est pourvu la mentonnière (8) est un clip (11).

6. Sous-ensemble constitué d'un oculaire semi-rigide (5) apte à être courbé, pourvu d'encoches latérales (9) permettant le montage de l'oculaire sur des pivots, et sur le pourtour duquel est fixée une coiffe (6) par une ligne de fixation étanche (12) contournant les encoches.

7. Sous-ensemble selon la revendication 6 dont l'oculaire comporte en partie basse un trou de fixation (10) contourné par la ligne de fixation (12).

## Claims

1. Protective helmet comprising a rigid frame (2) that pivots on two lateral pivots (3) of a headband (1), in such a way as to be able to be tilted towards the top to reveal the face or brought down into a service position, this frame determining a facial opening (4) and the helmet comprising an optical eyepiece (5) in front of the facial opening and a flexible cap (6) to cover the top of the head, a portion of the sides of the face and the bottom of the face, the cap being fixed to the optical eyepiece via a tight attachment (12) which substantially surrounds the optical eyepiece and the optical eyepiece having two lateral cutouts (9) for the mounting of the optical eyepiece on the lateral pivots (3) of the headband.

2. Protective helmet set forth in claim 1, of which the optical eyepiece (5) has in the lower portion a means of fastening (10) of the optical eyepiece to the lower portion (2b) of the frame.

3. Protective helmet set forth in claim 2 of which said means of fastening (10) is a hole.

4. Protective helmet set forth in claim 2 of which the lower portion (2b) of the frame comprises a chincup (8) provided with a means (11) for the fastening of the optical eyepiece.

5. Helmet according to claims 3 and 4 of which the means of fastening of which the chincup is provided (8) is a clip (11).

6. Subset comprised of a semi-rigid optical eyepiece (5) able to be curved, provided with lateral cutouts (9) allowing for the mounting of the optical eyepiece on pivots, and on the edges of which is fixed a cap (6) via a tight attachment line (12) bypassing the cutouts.

7. Subset set forth in claim 6 of which the optical eyepiece comprises in the lower portion a fastening hole (10) bypassed by the fastening line (12).

## Patentansprüche

1. Schutzhelm, der einen starren Rahmen (2) aufweist, der auf zwei seitlichen Drehzapfen (3) eines Kopfbands (1) schwenkt, so dass er hochgeklappt werden kann, um das Gesicht freizugeben, oder in die Gebrauchsposition heruntergeklappt werden kann, wobei dieser Rahmen eine Gesichtsöffnung (4) definiert und der Helm ein Visier (5) vor der Gesichtsöffnung und eine weiche Haube (6) aufweist, um den Oberkopf, einen Teil der Seiten des Gesichts und den unteren Teil des Gesichts zu bedecken, wobei die Haube durch eine dichte Befestigung (12), die im wesentlichen um das Visier herumführt, an dem Visier befestigt ist und das Visier zwei seitliche Einkerbungen (9) für die Montage des Visiers auf den seitlichen Drehzapfen (3) des Bands aufweist.

2. Schutzhelm nach Anspruch 1, dessen Visier (5) am unteren Teil ein Mittel zur Befestigung (10) des Visiers am unteren Teil (2b) des Rahmens aufweist.

3. Schutzhelm nach Anspruch 2, dessen Befestigungsmittel (10) ein Loch ist.

4. Schutzhelm nach Anspruch 2, dessen unterer Rahmenteil (2b) ein Kinnstück (8) umfasst, das mit einem Mittel (11) zur Befestigung des Visiers versehen ist.

5. Schutzhelm nach den Ansprüchen 3 und 4, dessen Befestigungsmittel, mit dem das Kinnstück (8) versehen ist, ein Clip (11) ist.

6. Teilanordnung, die aus einem biegbaren halbstarren Visier (5) besteht, das mit seitlichen Einkerbungen (9) versehen ist, welche die Montage des Visiers auf Drehzapfen ermöglichen, und an dessen Umfang durch eine dichte Befestigungslinie (12), die um die Einkerbungen herumführt, eine Haube (6) befestigt ist.

7. Teilanordnung nach Anspruch 6, deren Visier am unteren Teil ein Befestigungsloch (10) aufweist, um das die Befestigungslinie (12) herumführt.
